# EUROPEAN PATENT APPLICATION

(11) **EP 1 614 744 A1**
(43) Date of publication of application: **11.01.2006**
(21) Application number: 05013730.6
(22) Date of filing: 24.06.2005
(51) Int. Cl.: C12M 3/00

(54) **Cell tissue culture management method and system**

(30) Priority: 25.06.2004 JP 2004188895
(71) Applicant: Hitachi, Ltd., Tokyo 100-8280 (JP)
(72) Inventor: Kuno, Norihito, c/o Hitachi, Ltd., Chiyoda-ku Tokyo 100-8220 (JP)
(74) Representative: Strehl Schübel-Hopf & Partner

(57) **Abstract**

In order to detect the mis-operation of cells or tissues in a culturing step and other working steps to prevent erroneous use in treatment of cultured cell and tissues besides those of the cell donor, a genetic information, held by a cell or tissue, is used as a cell tissue information, which is inseparable from the cell tissue itself and enables individual identification of the cell donor, that is, identification of the origin of the cell or tissue subject to a culture process, and the cell tissue information before and after culturing are compared and collated.

## Description

### BACKGROUND OF THE INVENTION

### 1. Field of the Invention

This invention relates to a cell tissue culture management method and system for managing the respective steps from receiving to shipment of a cell tissue to be subject to the culture process.

### 2. Description of the Prior Art

In recent years, regenerative medicine techniques, by which a damaged or lost biological tissue or organ is restored utilizing regenerating or reconstructing tissues and cells by the proliferation and differentiation abilities of cells, have come to be noted as new treatment methods. In order to actually perform such treatment, cells or tissues must be sampled from a patient, and after selecting and separating the necessary cells or tissues from the sampled cells or tissues, the cells or tissues must be cultured, and regenerated or proliferated ex vivo the body to the enough amount for the treatment. Extremely troublesome operations, such as exchange of media and transfer of the cells or tissue to a different container, are required not only in the cell or tissue sampling step, separation step, and culturing step but also in a cell or tissue preservation step, transportation step, and inspection step. The troublesomeness of such working steps not only require time for the operations but also increase the possibilities of human errors, such as the mis-operation of the cells or tissues from different patients in a working step, that lead to treatment using amplified cells or tissue originating not from the original patient but from another person and thereby causing serious damage to the patient due to immunological rejection.

Numerous patents have been applied(since priorly) towards resolving the above issue. For example, among culture management devices for managing cell culture, there is an arrangement system, wherein an identification code is set for each biological sample and the culture records of the biological samples provided with the identification codes are stored to manage culturing while lightening the burden on workers regarding biological sample management in cell culture (see for example, Patent Document 1), and an arrangement system, wherein, in order to accurately and simply manage the association of a cell donor (patient) and cultured cells, cultured from the cells provided, and perform rapid collation of the cultured cells and the patient, containers, which are provided with identification information unique to the sampled cells, are used as carry-in containers, intermediate containers for culturing, and carry-out containers that are used in the cell culture to manage the cell culture by means of the identification information (see for example, Patent Document 2).

With the arts disclosed in the above-mentioned Patent Document 1 and Patent Document 2, the identification code or identification information for identifying each biological sample is stored in a biological information identification means that comprises a barcode, IC chip, or other non-biological storage medium that is attached to a container for processing or holding the biological sample. However, since such a cell or tissue identification information storage medium, that is formed of non-biological material, is separable from the biological sample itself, the correspondence of the identification code or identification information with the biological sample breaks down when the biological sample becomes mixed up due to human error by a worker or erroneous operation of a device, etc., thus causing the identification code provided on a container to become unmatched with the biological sample that is contained.

In order to resolve this problem, a method has been proposed wherein a biological information identification means, storing biological substance identification information, is mixed in a biological substance to be identified and the biological substance is identified by reading the information from the coexisting biological information identification means to prevent mixing up of the donor or recipient in the process of performing treatment or inspection using the biological substance (see, for example, Patent Document 3).

With this method, in comparison to the case where a non-biological matter, such as a barcode or IC chip, etc., is adhered as a storage medium onto a container, since the biological information identification means constantly coexists with the biological sample even though it is separable from the biological sample, the correspondence of the biological information identification means and the biological sample is maintained. Since this correspondence is maintained even upon transfer among containers, the risk of mis-operating biological samples due to human error, etc., can be reduced. However, in using the processed biological sample in treatment, etc., the trouble of separating the biological information identification means, which is a non-biological matter, from the biological sample is required, and the work, of guaranteeing that the biological information identification means has been removed completely, is complex and troublesome.

[Patent Document 1] Japanese Published Unexamined Patent Application No. 2002-269180 (Paragraphs 0007 to 0022, FIG. 1)

[Patent Document 2] Japanese Published Unexamined Patent Application No. 2004-119 (Paragraphs 0006 to 0041, FIG. 1)

[Patent Document 3] Japanese Published Unexamined Patent Application No. 2003-180662 (Paragraphs 0011 to 0029, FIG. 1)

With the above-described conventional arts, since the cell or tissue identification information storage medium, which is formed of non-biological matter, is separable from the biological sample, the possibility of a container and a contained biological sample becoming mixed up due to human error, etc., cannot be eliminated completely and consequently the correspondence between the identification information storage medium and the biological sample cannot be maintained.

Also, though the making of an identification information storage medium, which is a non-biological matter, coexist with a biological sample by mixing the storage medium in order to maintain the correspondence with the biological sample is effective in terms of keeping the correspondence, this gives rise to the new issue of having, from the standpoint of safety, to completely remove the identification information storage medium, which is a non-biological matter, in using the biological sample for actual treatment.

This invention has been made in view of the various circumstances described above, and a principal object thereof is to provide a cell tissue culture management method and system by which the mis-operation of cells and tissues in cell culture can be prevented.

### SUMMARY OF THE INVENTION

In order to achieve the above object, with the present invention, a genetic information, held in a cell or tissue, is used as a biological sample information (cell culture information), which is inseparable from the biological tissue (cell tissue) and enables individual identification of the cell donor, that is, identification of the origin of the cell or tissue subject to cell culture, and the genetic information before and after the cell culture process are compared and collated.

By this invention, mixing up of cells or tissues in cell culture can be prevented.

### BRIEF DESCRIPTION OF THE DRAWINGS

FIG. 1 is a process sequence diagram of a cell tissue culture management method of a first embodiment;
FIG. 2 is a block diagram showing the device arrangement of a cell tissue culture management system of the first embodiment;
FIG. 3 is a diagram showing an example of the data structure of an information storage unit (management DB) of the embodiment;
FIG. 4 is a diagram illustrating a case of using the information of five SNP positions as genetic information;
FIG. 5 is a block diagram illustrating, in regard to the first embodiment, the overall flow of information and objects that includes a hospital, the arrangement of a cell processing facility that executes a cell processing step, and the arrangement of the cell tissue culture management system;
FIG. 6 is a judgment flow in an information collating step of FIG. 1; and
FIG. 7 is a block diagram illustrating, in regard to a second embodiment, the overall flow of information and objects that includes a hospital, the arrangement of a cell processing facility that executes a cell processing step, and the arrangement of the cell tissue culture management system.

### DETAILED DESCRIPTION OF THE PREFERRED EMBODIMENTS

### First Embodiment

A first embodiment of a best mode for carrying out this invention's cell tissue culture management method and system will now be described in detail with reference to the drawings. [Process sequence of a cell tissue culture management method]

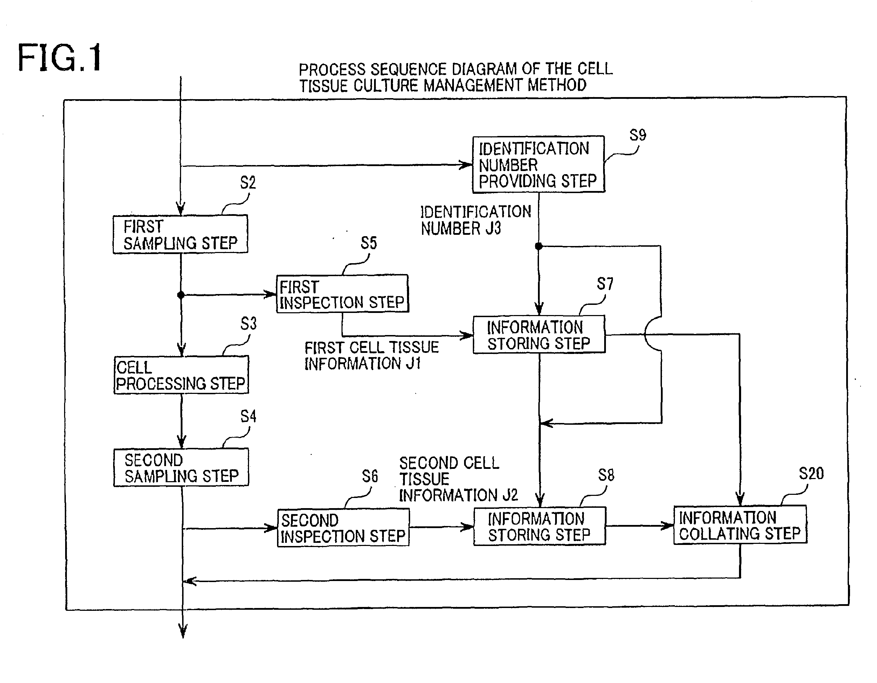

FIG. 1 is a process sequence diagram of the first embodiment's cell tissue culture management method. This process sequence will now be described with reference to FIG. 1.

As shown in FIG. 1, the process sequence comprises: a first sampling step S2 of sampling a sample (first cell tissue) for genetic information analysis of a cell or tissue (referred to hereinafter as "cell tissue"), which has been received and is to be subject to a culture process, from the cell tissue; a cell processing step S3 of subjecting the remaining cell tissue, among the cell tissue from which the sample for genetic information analysis has been sampled in first sampling step S2, to the culture process; and a second sampling step S4 of sampling, after the culture process of the cell tissue in cell processing step S3, a sample (second cell tissue) for genetic information analysis of the cell tissue that has been subject to the culture process. The process sequence furthermore comprises: a first inspection step S5 of performing genetic information analysis on the sample which is sampled in the above-mentioned first sampling step S2 and thereby obtaining a first cell tissue information J1; a second inspection step S6 of performing genetic information analysis on the sample which is sampled from the cell tissue after the culture process in the above-mentioned second sampling step S4 and thereby obtaining a second cell tissue information J2; an information storing step S7 of storing first cell tissue information J1, obtained in first inspection step S5, in association with an identification number J3; an information storing step S8 of storing second cell tissue information J2, obtained in second inspection step S6, in association with identification number J3; an identification number providing step S9 of providing identification number J3 to the received cell tissue to be subject to the culture process; and an information collating step S20 of collating first cell tissue information J1, obtained in first inspection step S5, and second cell tissue information J2, obtained in second inspection step S6, and examining the correspondence of the information.

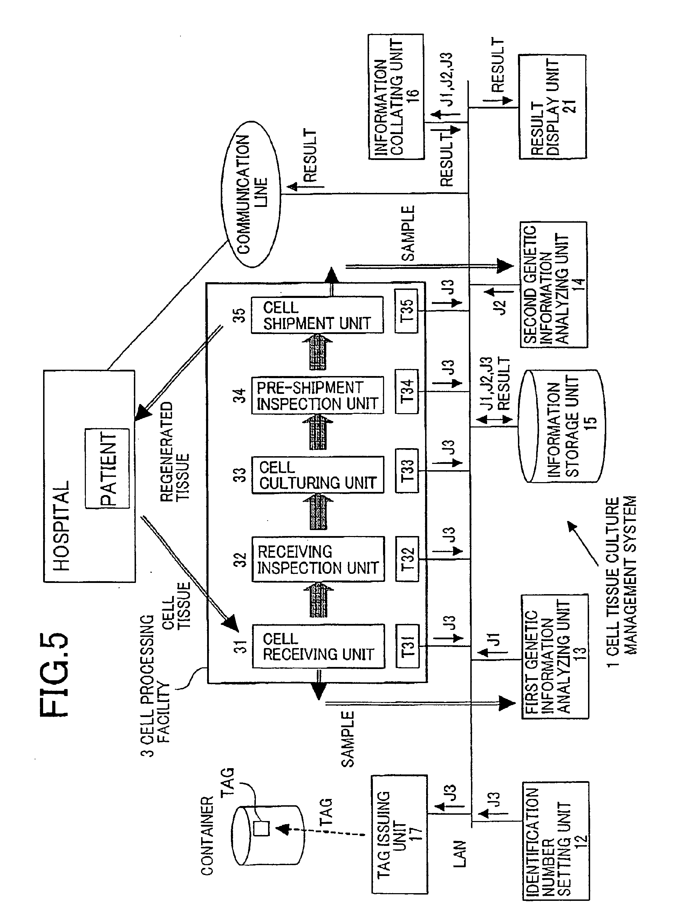

In the above-mentioned first sampling step S2, an enough amount of the sample to perform genetic information analysis and obtain first cell tissue information J1 is sampled from the cell tissue to be subject to the culture process. Also, in second sampling step S4, an enough amount of the sample to perform genetic information analysis and obtain second cell tissue information J2 is sampled from the cell tissue that has been subject to the culture process. Also, identification number J3, to be provided to the received cell tissue to be subject to the culture process, is stored in association with the first cell tissue information as mentioned above and is stored (written), for example, in a barcode, IC chip, or other tag adhered externally onto a container for containing the cell tissue (see FIG. 5).

### [Device composition of a cell tissue culture management system]

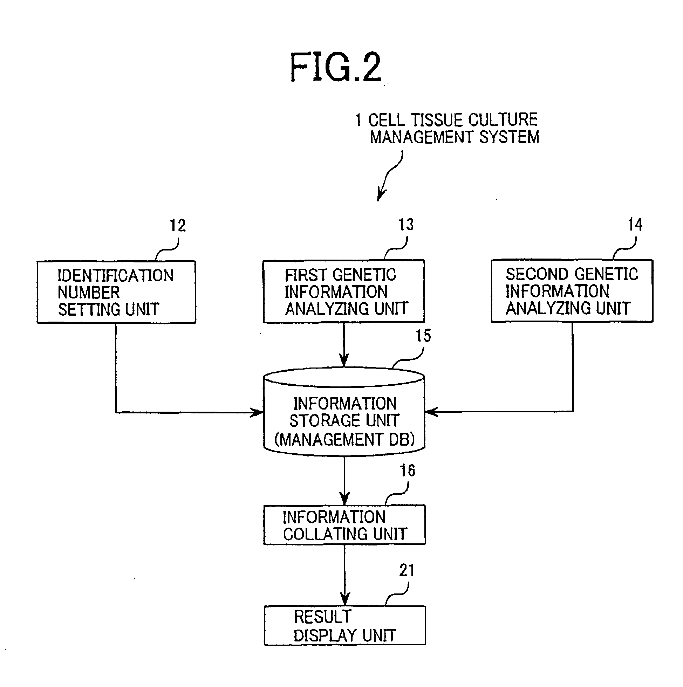

FIG. 2 is a block diagram showing the first embodiment's device composition of the cell tissue culture management system. The device composition of the cell tissue culture management system of the first embodiment will now be described with reference to FIG. 2 (and where suitable, FIG. 1). The devices, equipments, etc., that make up cell processing step S3 of FIG. 1 are not illustrated in FIG. 2.

As shown in FIG. 2, cell tissue culture management system 1 comprises an identification number setting unit 12, a first genetic information analyzing unit 13, a second genetic information analyzing unit 14, an information storage unit 15 (management DB) , an information collating unit 16, and a result display unit 21. A single genetic information analyzer (not shown) , having the function of performing the analysis of genetic information, may be used as first genetic information analyzing unit 13 and second genetic information analyzing unit 14. Also, a single computer (not shown), having a computing device, a storage device, and a display device, may be used as identification number setting unit 12, information storage unit 15, information collating unit 16, and result display unit 21. Obviously, these respective units 13 to 16 and 21 may be arranged as individually independent genetic information analyzers or computers. The sending and receiving of information and data among these genetic information analyzers and computers may be carried out online in a wired or wireless manner or offline using an information recording medium. In the description that follows, it will be deemed that the respective units 13 to 16 and 21 are composed as individually independent genetic information analyzers or computers that are connected to each other by a LAN (Local Area Network) (thus forming the cell tissue culture management system) . Though omitted from FIG. 2, a tag issuing unit 17, which issues tags, and other units to be described later (see FIG. 5) are also included in cell tissue culture management system 1 of the first embodiment.

In FIG. 2, first genetic information analyzing unit 13 has a function of obtaining first cell tissue information J1 by analyzing the genetic information of the cell tissue to be subject to the culture process, second genetic information analyzing unit 14 has a function of obtaining second cell tissue information J2 by analyzing the genetic information of the cell tissue that has been subject to the culture process, and the results obtained by these analysis units are stored in information storage unit 15. First cell tissue information J1 and second cell tissue information J2 (and furthermore, identification number J3) are stored in information storage unit 15, and a management DB (database) is constructed with these information.

Identification number setting unit 12 has a function of providing identification number J3 to the cell tissue to be subject to the culture process, and in the above-mentioned information storage unit 15, first cell tissue information J1 and second cell tissue information J2 are stored in association with this identification number J3.

Information collating unit 16 has a function of collating first cell tissue information J1 and second cell tissue information J2 and thereby detecting matching or non-matching. The detection result is displayed via result display unit 21. If first cell tissue information J1 and second cell tissue information J2 are matched, this means that there has been no mixing up, etc., in cell processing step S3 and cell culture has been carried out appropriately by the culture process.

At first genetic information analyzing unit 13, which performs inspection in first inspection step S5, at least one or a plural information among DNA base sequence information, single nucleotide polymorphism (SNP) information, microsatellite polymorphism information, and minisatellite polymorphism information is analyzed and the analysis result is sent to and saved in information storage unit 15 as first cell tissue information J1. And from the cell tissue that has been subject to the culture process (culture amplification process) in cell processing step S3, a cell tissue portion is sampled as a sample on which inspection in second inspection step S6 is to be performed using second genetic information analyzing unit 14. Here also, at least one or a plural information among DNA base sequence information, single nucleotide polymorphism (SNP) information, microsatellite polymorphism information, and minisatellite polymorphism information (that is, the information corresponding to that of first inspection step S5) is analyzed and the analysis result is sent to and saved in information storage unit 15 as second cell tissue information J2.

At information collating unit 16, first cell tissue information J1 and second cell tissue information J2, which have been saved in information storage unit 15, are read and collated to confirm, before and after the culture process in cell processing step S3, that the cell tissue subject to the process originates from the same cell donor (patient).

### [Data structure of the management DB and cell tissue information]

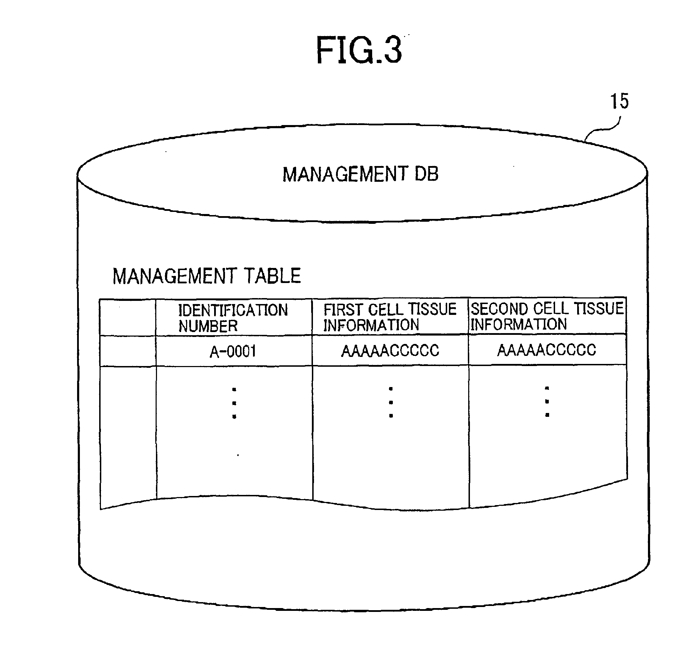

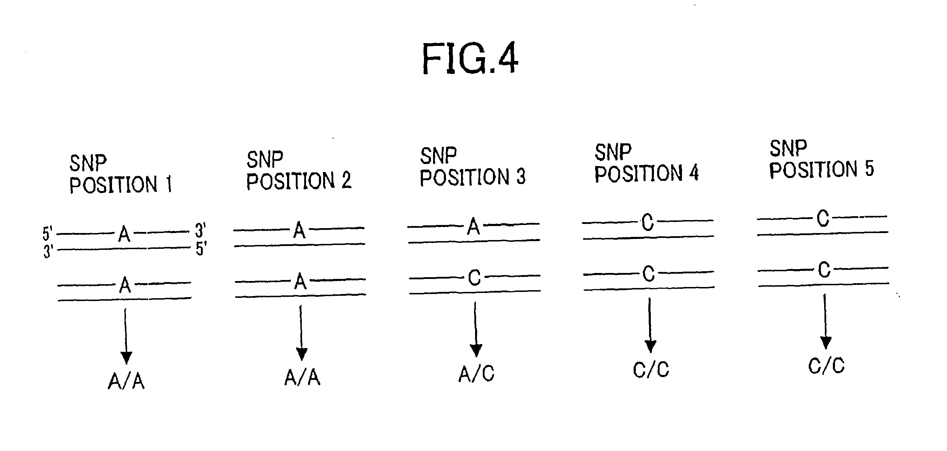

An example of the data structure of the management DB constructed in information storage unit 15 is shown in FIG. 3. Also, a case of using the information on five SNP positions as the genetic information is illustrated in FIG. 4. With reference to these FIGURES, an example of using the SNP information as the cell tissue information (first cell tissue information J1 and second cell tissue information J2) will now be described along with the data structure of the management DB.

In order to identify the cell tissue to be subject to the culture process, typing of SNPs of arbitrary positions and number is carried out using chromosomal DNA or mitochondrial DNA as the nucleic acid sample. In this process, collation with data in a DNA data bank, such as NCBI (National Center for Biotechnology Information), is carried out to specify the positions of the SNPs to be analyzed. Here a case of using five SNPs will be described. First, the sample that has been sampled from the cell tissue to be subject to the culture process in first sampling step S2 is inspected and analyzed in first inspection step S5. If as a result of analyzing the respective genotypes of the first to the fifth SNP positions in first inspection step S5, it is found that the first is A/A, the second is A/A, the third is A/C, the fourth is C/C, and the fifth is C/C, "AAAAACCCCC" is stored as first cell tissue information J1 in association with identification number J3, which is "A-0001," in the management DB (information storage unit 15) by first genetic information analyzing unit 13. The above-mentioned first, second, and fifth SNPs are homotypes at the alleles and the above-mentioned third and fourth SNPs are heterotypes at the alleles.

In first inspection step S5 for obtaining first cell tissue information J1, the number of SNPs typing of chromosomal DNA or mitochondrial DNA may be set to any suitable number from 1 to N and is preferably set to 10 or more.

In likewise manner, in second inspection step S6, second genetic information analyzing unit 14 inspects and analyzes the sample obtained in second sampling step S4 from the cell tissue after the culture process. Second cell tissue information J2 is thereby obtained. If there was no mixing up of cell tissue or other problems, the analysis results of the respective genotypes of the SNP positions that are obtained should be the same as the results obtained in first inspection step S5, that is, the genotype of the first position should be A/A, that of the second position should be A/A, that of the third position should be A/C, that of the fourth position should be C/C, and that of the fifth position should be C/C. Consequently, second genetic information analyzing unit 14 stores "AAAAACCCCC" as second cell tissue information J2 in association with identification number J3, which is "A-0001," in the management DB. At information collating unit 16, first cell tissue information J1, "AAAAACCCCC," and second cell tissue information J2, "AAAAACCCCC," which are associated with identification number J3, "A-0001," are collated to compare and collate whether or not these are the same information (detect matching or non-matching).

### [Overall flow, etc.]

FIG. 5 is a block diagram illustrating, in regard to the first embodiment, the overall flow of information and objects that includes a hospital, the arrangement of a cell processing facility that executes the cell processing step, and the composition of the cell tissue culture management system. In FIG. 5, cell processing facility 3 is a facility in which cell culture is carried out and has a cell receiving unit 31, a receiving inspection unit 32, a cell culture unit 33, a pre-shipment inspection unit 34, and a cell shipping unit 35. The respective units 31 to 35 are equipped with terminals T31 to T35 for reading the information in tags adhered onto containers and sending the information to information storage unit 15. The respective units 31 to 35 are devices or equipments for executing cell processing step S3 of FIG. 1. Terminals T31 to T35 are terminals for tracking the respective steps of cell processing step S3 in which cell culture is carried out. Also, though not illustrated in FIG. 2, symbol 17 indicates a tag issuing unit that issues tags (wireless IC tags or barcodes) , each of which is adhered onto a container in which a cell tissue is contained and in each of which an identification number J3 is stored (written). The respective units of symbols 12 to 16 and 21 are as has been described above and redundant description will be omitted.

The flow of the respective processes of FIG. 5 will now be described (with reference to FIG. 1, etc., where suitable). The cell tissue to be subject to the culture process, which is obtained from a patient at a hospital, is sent from the hospital to cell processing facility 3. At this cell processing facility 3, a portion of the cell tissue received at cell receiving unit 31 is sampled for inspection in the above-mentioned first inspection step S5 (first sampling step S2) and the remaining portion is contained in a container for cell culture. At identification number setting unit 12, a unique identification number J3 is issued. Identification number J3 is stored in a tag by tag issuing unit 17 and is adhered onto the container containing the cell tissue. The information of the tag that is adhered onto this container is read by the respective terminals T31 to T35 provided in the respective units 31 to 35.

To be more specific, at cell receiving unit 31, the sampling of the sample for inspection in first inspection step S5 from the received cell tissue (first sampling step S2), the placing of the remaining cell tissue in the container, the issuing of identification number J3, the issuing of the tag in which identification number J3 is stored, and the adhesion of the tag to the container are carried out. Then from terminal T31 to information storage unit 15, an ID unique to terminal T31, identification number J3, and the time (time of reading) are sent and these are stored in information storage unit 15. The ID unique to terminal T31 is information that specifies the step that is carried out on the cell tissue (the same applies hereinafter).

First cell tissue information J1, which is the result of inspection in first inspection step S5 (first genetic information analyzing unit 13), is sent from first genetic information analyzing unit 13 to information storage unit 15 and stored in association with identification number J3 (information storing step S7; see FIG. 3).

At receiving inspection unit 32 that follows, a predetermined inspection (inspection of contamination due to viruses, bacteria, etc.) is carried out on the cell tissue by an unillustrated inspection device. Here also,theinformation in the tag adhered to the container is read by terminal T32. Then from terminal T32 to information storage unit 15, an ID unique to terminal T32, identification number J3, and the time (time of reading) are sent and these are stored in information storage unit 15.

At cell culture unit 33 that follows, cell culture (amplification) is carried out by an unillustrated cell culture device. Here also, the information in the tag adhered to the container is read by terminal T33. Then from terminal T33 to information storage unit 15, an ID unique to terminal T33, identification number J3, and the time (time of reading) are sent and these are stored in information storage unit 15.

At pre-shipment inspection unit 34 that follows, a predetermined inspection (quality inspection) is carried out on the cultured cell tissue by an unillustrated inspection device. Here also, the information in the tag adhered to the container is read by terminal T34. Then from terminal T34 to information storage unit 15, an ID unique to terminal T34, identification number J3, and the time (time of reading) are sent and these are stored in information storage unit 15.

At cell shipping unit 35 that follows, the shipment of the cultured cell tissue (regenerated tissue) is carried out by an unillustrated shipping device. In the process of shipping, a sample for inspection in second inspection step S6 is sampled from the cell tissue (second sampling step S4). The sampled cell tissue is then inspected in second inspection step S6. Second cell tissue information J2, which is the result of inspection in second inspection step S6 (second genetic information analyzing unit 14), is sent from second genetic information analyzing unit 14 to information storage unit 15 and stored in association with identification number J3 (information storing step S8; see FIG. 3).

Information collation step S20 is then carried out by information collating unit 16.

### [Flow of judgment in the information collating step]

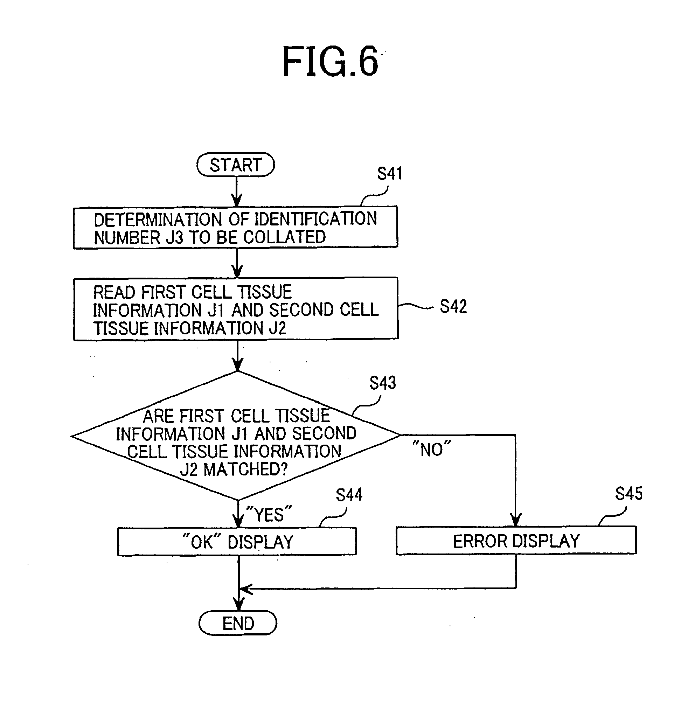

Information collation step S20, which is executed by information collating unit 16, will now be described with reference to a flowchart. FIG. 6 shows the judgment flow in the information collating step. In FIG. 6, information collating unit 16 first determines the identification number J3 of the cell tissue on which collation is to be performed. That is, the identification number J3 to be collated is determined (S41). This determination of identification number J3 may be carried out by reading the information in the tag adhered to a container that is waiting for shipment at cell shipping unit 35 by terminal T35. Identification number J3 may also be determined by manual input from an unillustrated keyboard, or identification number J3 may be acquired via a LAN or a communication line. First cell tissue information J1 and second cell tissue information J2 that are associated with identification number J3 (forexample, "A-0001"), for which it has been determined that collation is to be performed, are then read from information storage unit 15 (S42). Whether or not the read first cell tissue information J1 and second cell tissue information J2 match is then judged by comparison and collation (matching or non-matching is detected) (S43).

If there has not been a mixing up of cell tissues due to human error, erroneous operation of a device, etc., in cell processing step S3, first cell tissue information J1 and second cell tissue information J2, which are collated here, should match.

If first cell tissue information J1 and second cell tissue information J2 match, an "OK" display is displayed on an unillustrated monitor via result display unit 21 (S44). If first cell tissue information J1 and second cell tissue information J2 do not match, since it can then be assumed that mixing up of cell tissues, etc., occurred due to some cause in the cell processing step, an error display is displayed via result display unit 21 to prevent use of the processed cell tissue in subsequent treatment, etc., (S45). It can thus be confirmed before and after the culture process that the cell tissue subject to the culture process in cell processing step S3 originates from the same cell tissue donor (patient) and the problem of mixing up of cell tissues can be resolved. The result of collation by information collating unit 16 is arranged so that it can be viewed at the hospital via the communication line.

With the above-described first embodiment, since collation is carried out using genetic information (first cell tissue information J1 and second cell tissue information J2) unique to the cell tissue, the mis-operation of cell tissue can be discovered and prevented without fail. Also, since a unique identification number J3 is provided to the received cell tissue and reading and storage of identification number J3 are carried out in the respective steps, the processing record of the cell tissue can be left without fail and processing record management can be carried out without fail.

### Second Embodiment

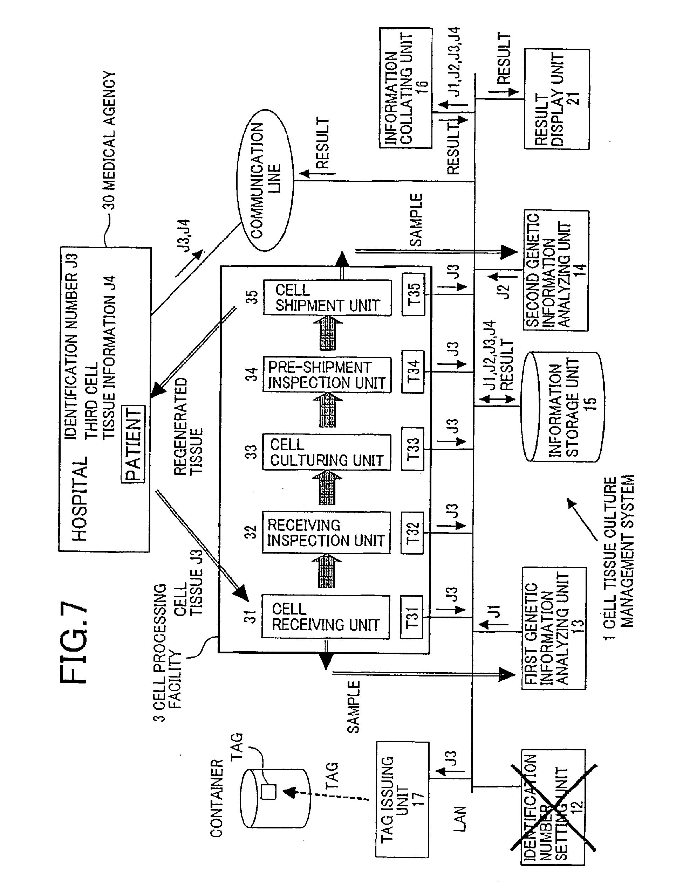

A second embodiment of the best mode for carrying out this invention's cell tissue culture management method and system will now be described in detail with reference to FIG. 7 (and with reference to FIG. 1 where suitable). FIG. 7 is a block diagram illustrating, in regard to the second embodiment, the overall flow of information and objects that includes a hospital, the arrangement of a cell processing facility that executes the cell processing step, and the composition of the cell tissue culture management system. Blocks provided with the same numbers as the blocks in FIG. 5 are the same as those shown in FIG. 5 and description thereof will be omitted.

This second embodiment differs from the first embodiment in that the setting of identification number J3 is carried out in the hospital or other medical agency 30 in which the cell tissue is sampled from the cell donor (and thus identification number setting unit 12 is unnecessary), the analysis of cell tissue information (third cell tissue information J4) on the cell tissue is also carried out at medical agency 30, and collation is performed with the incorporation of third cell tissue information J4, etc.

In FIG. 7, in the process of sending the cell tissue to cell processing facility 3, medical agency 30 analyzes third cell tissue information J4 separately from cell processing facility 3 (analyzes using a separate sample) and also sets identification number J3. The cell tissue is then transported, with identification number J3 being indicated, etc., to cell processing facility 3. In conjunction, the pair of identification number J3 and third cell tissue information J4 are sent via the communication line.

By the above, cell processing facility 3 (cell tissue culture management system 1) can perform, in addition to the collation of the first embodiment, a collation that incorporates third cell tissue information J4, provided from medical agency 30, at collating unit 16. That is, third cell tissue information J4, which has been obtained separately, can be acquired to detect the matching or non-matching of cell tissue by performing at least one of either of the collation of first cell tissue information J1 with third cell tissue information J4 and the collation of second cell tissue information J2 with third cell tissue information J4. Thus in addition to the prevention of mis-operation in cell processing facility 3, the prevention of mis-operation, etc., during transport of the cell tissue from medical agency 30 to cell processing facility 3 is also enabled.

Furthermore, in the process of shipping the cell tissue (regenerated tissue) after the culture process to medical agency 30, by sending, etc., via the communication line, at least one of either of first cell tissue information J1 and second cell tissue information J2 along with identification number J3 to medical agency 30, the validity of the regenerated tissue can be verified at medical agency 30 to further prevent mis-operation. As the sample for obtaining third cell tissue information J4, a cell tissue, etc., of any portion of the patient may be used.

### Other Embodiments

This invention, which has been described above, is not limited to the above-described embodiments and can be modified widely within the scope of the spirit of the art thereof.

For example, though first inspection step S5 is performed at the point of receiving and second inspection step S6 is performed at the point of shipping in the embodiments described above, this invention is not limited thereto and inspections may be carried out at intermediate stages. Also (see FIG. 5) , for example, the contents (culturing temperature, culturing time, etc.) of the processes carried out in cell culture unit 33 may be associated with identification number J3 and stored in information storage unit 15 via terminal T33. That is, a procedure of associating identification number J3 with the information on the steps carried out on the cell tissue in cell processing step S3 and storing this information in information storage unit 15 may be carried out.

Also for example, in the first embodiment, first cell tissue information J1 may serve in common as identification number J3. That is, first cell tissue information J1 may be used as identification number J3. In this case, there is no need to provide identification number setting unit 12 in particular. Also, the genetic information used for identification is not limited to any specific information. Also, the collation in information collating step S20 (information collating unit 16) is not limited to any specific form in particular and, for example, the collation may be performed in the form of performing image analysis, etc., and performing pattern matching.

Also for example, in the process of acquiring first cell tissue information J1 and second cell tissue information J2, an SNP search may carried out using a marker (for example, a telomere or a microsatellite, etc. ) on DNA as a starting point.

Also with the second embodiment, third cell tissue information J3 may be obtained by genetic analysis at a third party agency that is neither medical agency 30, which is to perform transplantation, nor cell processing facility 3, which is to perform culturing.

## Claims

1. A cell tissue culture management method comprising:
a first sampling step of sampling a first cell tissue from a cell tissue to be subject to a culture process;
a first inspection step of obtaining a first cell tissue information, which is a genetic information on the first cell tissue;
a cell processing step of subjecting the cell tissue, to be subject to the culture process, to the culture process;
a second sampling step of sampling a second cell tissue from the cell tissue that has been subject to the culture process;
a second inspection step of obtaining a second cell tissue information, which is a genetic information on the second cell tissue; and
an information collating step of collating the first cell tissue information and the second cell tissue information to detect the matching or non-matching of the cell tissues.

2. The cell tissue culture management method according to Claim 1, further comprising:
a step of adding an identification number to the cell tissue to be subject to the culture process;
a step of storing the first cell tissue information in association with the identification number; and
a step of managing the cell processing step in accordance with the associated identification number.

3. A cell tissue culture management method of managing a cell processing step, in which a cell tissue is subject to a culture process and comprising at least one step between receiving of the cell tissue to be subj ect to the culture process and shipment after the culture process, by means of a cell tissue culture management system, comprising a computing device and a storage device, wherein
the cell tissue culture management system executes:
a procedure of acquiring a first cell tissue information, which is a genetic information on the cell tissue that is obtained in a first inspection step;
a procedure of acquiring a second cell tissue information, which is a genetic information on a cell tissue that is obtained in a second inspection step carried out after execution of at least one step in the cell processing step; and
a procedure of collating the first cell tissue information and the second cell tissue information to detect the matching or non-matching of the cell tissues.

4. The cell tissue culture management method according to Claim 3, wherein, in the case of providing a unique identification number to the cell tissue to manage the cell processing step,
the cell tissue culture management system executes a procedure of storing the correspondence between the first cell tissue information and the identification number in the storage device, and
executes at least one procedure among the following procedures A, B, and C:
(A) a procedure of storing an information, specifying the steps carried out on the cell tissue in the cell processing step, in association with the identification number in the storage device;
(B) a procedure of storing an information, concerning the processes carried out on the cell tissue in the cell processing step, in association with the identification number in the storage device;
(C) a procedure of indicating the identification number on a container containing the cell tissue and managing the cell processing step using the identification number as an identification information on the cell tissue.

5. The cell tissue culture management method according to anyone of Claims 1 to 4, wherein the first cell tissue information and the second cell tissue information, which are genetic information on the cell tissues, are DNA base sequence information.

6. The cell tissue culture management method according to any one of Claims 1 to 4, wherein the first cell tissue information and the second cell tissue information, which are genetic information on the cell tissues, are single nucleotide polymorphism (SNP) information.

7. The cell tissue culture management method according to any one of Claims 1 to 4, wherein the first cell tissue information and the second cell tissue information, which are genetic information on the cell tissues, are gene microsatellite polymorphism or minisatellite polymorphism information.

8. The cell tissue culture management method according to any one of Claims 1 to 7, wherein the first inspection step is carried out at the time of receipt of the cell tissue to be subject to the culture process and the second inspection step is carried out at the time of shipment of the cell tissue that has been subject to the culture process.

9. The cell tissue culture management method according to any one of Claims 2 and 4 to 8, wherein the identification number is stored in a storage unit equipped in a container containing the cell tissue.

10. The cell tissue culture management method according to any one of Claims 1 to 9, wherein
a third cell tissue information, which is obtained separately, is acquired for the cell tissue, and
at least one of either of collation of the first cell tissue information and the third cell tissue information and collation of the second cell tissue information and the third cell tissue information is carried out to detect the matching or non-matching of the cell tissues.

11. A cell tissue culture management system for managing a cell processing step, wherein a cell tissue is subject to the culture process and comprising at least one step between receiving of the cell tissue to be subject to the culture process and shipment after the culture process, comprising:
a first genetic information analyzing unit, analyzing a first cell tissue information, which is genetic information obtained from the cell tissue to be subject to the culture process;
a second genetic information analyzing unit, analyzing a second cell tissue information, which is genetic information obtained from a cell tissue resulting from the culture process of the first cell tissue; and
an information collating unit, collating the first cell tissue information and the second cell tissue information to detect matching or non-matching.

12. The cell tissue culture management system according to Claim 11, further comprising:
an identification number setting unit, providing an identification number to the cell tissue to be subject to the culture process;
the information storage unit, storing the first cell tissue information in association with the identification number; and
an information collating unit, collating the second cell tissue information and the first cell tissue information to detect matching or non-matching.

13. The cell tissue culture management system according to Claim 11, further comprising
an arrangement that displays and notifies to the exterior the matching or non-matching detected by the information collating unit.

14. The cell tissue culture management system according to any one of Claims 11 to 13, further comprising:
a communicating unit, transmitting in a wired or wireless manner, the identification number provided by the identification number setting unit, the analysis result from the first genetic information analyzing unit, and the analysis result from the second genetic information analyzing unit, to the information collating unit.

15. The cell tissue culture management system according to any one of Claims 11 to 14, wherein
the information collating unit has an arrangement of performing collation by incorporating a third cell tissue information that is obtained separately for the cell tissue.
